(19) Europäisches Patentamt
European Patent Office
Office européen des brevets

(11) **EP 3 868 468 B1**

(12) **EUROPEAN PATENT SPECIFICATION**

(45) Date of publication and mention
of the grant of the patent:
**27.08.2025 Bulletin 2025/35**

(21) Application number: **19873017.8**

(22) Date of filing: **14.10.2019**

(51) International Patent Classification (IPC):
**B01J 23/78** $^{(2006.01)}$        **B01J 23/88** $^{(2006.01)}$
**C07C 5/333** $^{(2006.01)}$

(52) Cooperative Patent Classification (CPC):
(C-Sets available)
**B01J 23/002; B01J 23/881; B01J 35/70;**
**B01J 37/0018; B01J 37/088; C07C 5/3332;**
B01J 2235/15; B01J 2523/00; C07C 2523/78;
C07C 2523/887; Y02P 20/52        (Cont.)

(86) International application number:
**PCT/CN2019/110935**

(87) International publication number:
**WO 2020/078303 (23.04.2020 Gazette 2020/17)**

(54) **CATALYST FOR DEHYDROGENATION OF ALKYL AROMATIC HYDROCARBON AND PREPARATION METHOD THEREFOR**

KATALYSATOR ZUR DEHYDRIERUNG VON ALKYLAROMATISCHEN KOHLENWASSERSTOFFEN UND VERFAHREN ZUR HERSTELLUNG DAVON

CATALYSEUR POUR LA DÉSHYDROGÉNATION D'UN HYDROCARBURE AROMATIQUE D'ALKYLE ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.10.2018 CN 201811201536**
**16.10.2018 CN 201811201422**

(43) Date of publication of application:
**25.08.2021 Bulletin 2021/34**

(73) Proprietors:
• **China Petroleum & Chemical Corporation**
**Beijing 100728 (CN)**
• **Shanghai Research Institute of Petrochemical Technology, SINOPEC**
**Shanghai 201208 (CN)**

(72) Inventors:
• **MIAO, Changxi**
**Shanghai 201208 (CN)**
• **WEI, Chunling**
**Shanghai 201208 (CN)**
• **SONG, Lei**
**Shanghai 201208 (CN)**
• **CHEN, Tong**
**Shanghai 201208 (CN)**
• **NI, Junping**
**Shanghai 201208 (CN)**

(74) Representative: **Hoffmann Eitle**
**Patent- und Rechtsanwälte PartmbB**
**Arabellastraße 30**
**81925 München (DE)**

(56) References cited:
WO-A1-2015/169825    CN-A- 1 490 084
CN-A- 1 810 370      CN-A- 103 372 452
CN-A- 105 080 559    CN-A- 107 790 150
CN-A- 108 203 365    DE-A1- 4 025 930

(52)  Cooperative Patent Classification (CPC): (Cont.)

C-Sets
**C07C 5/3332, C07C 15/46;**
B01J 2523/00, B01J 2523/12, B01J 2523/13,
B01J 2523/23, B01J 2523/3712, B01J 2523/68,
B01J 2523/842

**Description**

**Technical Field of the Disclosure**

The present disclosure relates to a catalyst for dehydrogenation of alkyl aromatic hydrocarbons and a method therefor, in particular to a catalyst comprising a compound containing iron and potassium, and a method therefor, and belongs to the field of dehydrogenation of alkyl aromatic hydrocarbons.

**Background of the Disclosure**

The industrial production method of alkenyl aromatic hydrocarbons is mainly dehydrogenation of alkyl aromatic hydrocarbons. For example, the industrial production method of styrene is mainly catalytic dehydrogenation of ethylbenzene, and its production capacity accounts for about 85% of the total production capacity of styrene. One of the keys of the method is catalyst for dehydrogenation of ethylbenzene to styrene. At present, basic components of catalyst for dehydrogenation of ethylbenzene to styrene in industry include a main catalyst, a cocatalyst, a pore-forming agent, and a binder, etc. Early catalysts are Fe-K-Cr system catalysts, such as the published US Patent 4467046 (Dehydrogenation catalyst) and European Patent 0296285A1 (A dehydrogenation catalyst having improved moisture stability and a process for making the same). Although such catalysts have good activity and stability, they have been gradually eliminated because the catalysts contain oxides of Cr, which would cause certain pollution to the environment. Later, the catalysts evolved into Fe-K-Ce-Mo system catalysts, in which Cr is replaced with Ce and Mo, which can better improve catalyst activity and stability while overcoming the disadvantages of Cr's high toxicity and pollution to the environment.

CN 103 372 452 A describes a catalyst for preparing styrene by dehydrogenation of ethylbenzene and preparation method thereof.

In catalysts for production of alkenyl aromatic hydrocarbons by means of dehydrogenation of alkyl aromatic hydrocarbons, iron oxide is a main catalyst, and potassium is a main cocatalyst. The addition of potassium can increase catalyst activity by more than one order of magnitude. For Fe-K-Ce-Mo catalysts, after high temperature calcination, the catalysts generally contain an $\alpha$-$Fe_2O_3$ phase and phases of a compound containing iron and potassium. A large number of research results show that a compound of iron and potassium is a main active phase or a precursor of active phase of catalysts for dehydrogenation of alkyl aromatic hydrocarbons. Therefore, the formation and structure of a compound of iron and potassium are of great significance to catalyst activity.

How to easily obtain required catalyst phases and structures so as to further improve catalyst activity has always been a topic of interest to researchers.

**Summary of the Disclosure**

A method for preparing a catalyst for dehydrogenation of an alkyl aromatic hydrocarbon according to the invention, as well as a method for dehydrogenation of an alkyl aromatic hydrocarbon are defined in the claims.

A first technical problem to be solved by the present disclosure is the technical problem of insufficient catalyst activity in the prior art. The present disclosure provides a new catalyst for the dehydrogenation of an alkyl aromatic hydrocarbon. The catalyst includes a compound containing iron and potassium. The compound containing iron and potassium has a special X-ray diffraction (XRD) pattern. The catalyst has the characteristic of high activity.

A second technical problem to be solved by the present disclosure is to provide a preparing method corresponding to the catalyst that solves the first technical problem.

A third technical problem to be solved by the present disclosure is to apply the catalyst, which solves the first technical problem above, to a method for dehydrogenation of an alkyl aromatic hydrocarbon.

In order to solve the first technical problem above, the present disclosure provides a catalyst for dehydrogenation of an alkyl aromatic hydrocarbon. The catalyst includes a compound containing iron and potassium. The compound containing iron and potassium consists of a $K_2Fe_{10}O_{16}$ phase and a $K_2Fe_{22}O_{34}$ phase.

According to a preferred implementation of the present disclosure, the compound containing iron and potassium has an X-ray diffraction (XRD) pattern as shown in the following table:

| 2θ (°) | d- Distance (Å) | Relative Intensity ($I/I_0 \times 100$) |
|---|---|---|
| 7.355±0.04 | 12.010±0.07 | VS |
| 14.752±0.03 | 6.000±0.03 | S |
| 18.825±0.16 | 4.710±0.05 | W |
| 19.846±0.07 | 4.470±0.03 | W |

(continued)

| 2θ (°) | d- Distance (Å) | Relative Intensity ($I/I_0 \times 100$) |
|---|---|---|
| 29.837±0.07 | 2.992±0.04 | S |
| 31.566±0.16 | 2.832±0.03 | M |
| 32.460±0.07 | 2.756±0.02 | W |
| 33.705±0.14 | 2.657±0.03 | S |
| 34.854±0.05 | 2.572±0.02 | M |
| 37.767±0.10 | 2.380±0.02 | M |
| 41.825±0.10 | 2.158±0.02 | W |
| 43.253±0.13 | 2.090±0.03 | M |
| 44.232±0.18 | 2.046±0.03 | W |
| 62.351±0.15 | 1.488±0.02 | M |

According to a preferred implementation of the present disclosure, the X-ray diffraction pattern further comprises X-ray diffraction peaks as shown in the following table:

| 2θ (°) | d- Distance (Å) | Relative Intensity ($I/I_0 \times 100$) |
|---|---|---|
| 17.868±0.08 | 4.960±0.03 | W |
| 30.346±0.03 | 2.943±0.02 | M |
| 36.342±0.11 | 2.470±0.03 | W-M |
| 39.929±0.18 | 2.256±0.03 | W |
| 53.921±0.16 | 1.699±0.03 | M |
| 55.549±0.06 | 1.653±0.02 | W |
| 63.154±0.07 | 1.471±0.02 | W |

According to a preferred implementation of the present disclosure, a mass ratio of the $K_2Fe_{10}O_{16}$ phase to the $K_2Fe_{22}O_{34}$ phase in the compound containing iron and potassium is in a range from 1.1 to 3.3, preferably in a range from 1.2 to 2.0.

According to a preferred embodiment of the present disclosure, the catalyst does not contain a free $\alpha$-$Fe_2O_3$ phase.

According to a preferred embodiment of the present disclosure, the catalyst includes the following components in weight percentage: (a) 65% to 80% of $Fe_2O_3$; (b) 6% to 14% of $K_2O$; (c) 9% to 13.5% of $CeO_2$; (d) 0.5% to 5% of $MoO_3$; and (e) 0.2% to 5% of CaO.

In order to solve the second technical problem above, the present disclosure provides a method for preparing a catalyst for dehydrogenation of an alkyl aromatic hydrocarbon, the method including the following steps of:

1) dry-mixing a first part of a potassium source with an iron source, a cerium source, a molybdenum source, a calcium source, and a pore-forming agent so as to obtain a catalyst precursor I; and
2) dissolving a second part of the potassium source with water, and adding the catalyst precursor I to obtain an as-made catalyst,

the catalyst including the following components in weight percentage: (a) 65% to 80% of $Fe_2O_3$; (b) 6% to 14% of $K_2O$; (c) 9% to 13.5% of $CeO_2$; (d) 0.5% to 5% of $MoO_3$; and (e) 0.2% to 5% of CaO; the catalyst does not contain a free $\alpha$-$Fe_2O_3$ phase,
wherein by amount of contained $K_2O$, a sum of a weight of the first part of the potassium source and a weight of the second part of the potassium source is a total weight of a required amount of potassium source, and the weight of the first part of the potassium source accounts for 60% to 90% of the total weight of the required amount of potassium source.

According to a preferred implementation of the present disclosure, in the step 1), adding the iron source in the form of at least one of red iron oxide and yellow iron oxide; adding the first part of the potassium source in the form of at least one of a potassium salt and potassium hydroxide; adding the cerium source in the form of a cerium salt; adding the molybdenum source in the form of at least one of a molybdenum salt and an oxide of molybdenum; and adding the calcium source in the form of at least one of a calcium salt, calcium oxide, and calcium hydroxide.

According to a preferred implementation of the present disclosure, in the step 2), adding the second part of the potassium source in the form of at least one of potassium hydroxide and potassium carbonate.

According to a preferred implementation of the present disclosure, an amount of addition of water in step 2) is not particularly limited. Those skilled in the art can reasonably control the humidity according to the requirement for extrusion. For example, the amount of addition of water accounts for, but not limited to, 18% to 32% of the total weight of the catalyst raw materials. Preferably, the water is deionized water.

According to a preferred implementation of the present disclosure, the pore-forming agent is a combustible material known to those in the art. For example, the pore-forming agent can be selected from sodium carboxymethyl cellulose, polymethylstyrene microspheres, methyl cellulose, hydroxyethyl cellulose, sesbania powder, graphite, etc. An amount of addition of the pore-forming agent accounts for 2.1% to 5.8% of the total weight of the catalyst.

According to a preferred implementation of the present disclosure, the method further includes following step of: 3) performing processes of wet-kneading, extruding, forming, drying, and calcinating on the as-made catalyst obtained in the step 2).

According to a preferred implementation of the present disclosure, in the step 3), a drying temperature is in a range from 45°C to 130°C, and a drying time is in a range from 4 to 24 hours.

According to a preferred embodiment of the present disclosure, in the step 3), calcinating is performed at 200°C to 400°C for 5 to 12 hours, and then at 750°C to 950°C for 3 to 8 hours.

According to a preferred implementation of the present disclosure, the as-prepared catalyst includes a compound containing iron and potassium, the compound containing iron and potassium consisting of a $K_2Fe_{10}O_{16}$ phase and a $K_2Fe_{22}O_{34}$ phase.

According to a preferred implementation of the present disclosure, the compound containing iron and potassium has an X-ray diffraction (XRD) pattern as shown in the following table:

| $2\theta$ (°) | d- Distance (Å) | Relative Intensity ($I/I_0 \times 100$) |
|---|---|---|
| 7.355±0.04 | 12.010±0.07 | VS |
| 14.752±0.03 | 6.000±0.03 | S |
| 18.825±0.16 | 4.710±0.05 | W |
| 19.846±0.07 | 4.470±0.03 | W |
| 29.837±0.07 | 2.992±0.04 | S |
| 31.566±0.16 | 2.832±0.03 | M |
| 32.460±0.07 | 2.756±0.02 | W |
| 33.705±0.14 | 2.657±0.03 | S |
| 34.854±0.05 | 2.572±0.02 | M |
| 37.767±0.10 | 2.380±0.02 | M |
| 41.825±0.10 | 2.158±0.02 | W |
| 43.253±0.13 | 2.090±0.03 | M |
| 44.232±0.18 | 2.046±0.03 | W |
| 62.351±0.15 | 1.488±0.02 | M |

According to a preferred implementation of the present disclosure, the X-ray diffraction pattern further includes X-ray diffraction peaks as shown in the following table:

| $2\theta$ (°) | d- Distance (Å) | Relative Intensity ($I/I_0 \times 100$) |
|---|---|---|
| 17.868±0.08 | 4.960±0.03 | W |
| 30.346±0.03 | 2.943±0.02 | M |

(continued)

| 2θ (°) | d- Distance (Å) | Relative Intensity (I/I$_0$ × 100) |
|---|---|---|
| 36.342±0.11 | 2.470±0.03 | W-M |
| 39.929±0.18 | 2.256±0.03 | W |
| 53.921±0.16 | 1.699±0.03 | M |
| 55.549±0.06 | 1.653±0.02 | W |
| 63.154±0.07 | 1.471±0.02 | W |

According to a preferred implementation of the present disclosure, a mass ratio of the $K_2Fe_{10}O_{16}$ phase to the $K_2Fe_{22}O_{34}$ phase in the compound containing iron and potassium is in a range from 1.1 to 3.3, preferably in a range from 1.2 to 2.0.

Catalyst particles prepared in the present disclosure can be of various shapes, such as a solid cylindrical shape, a hollow cylindrical shape, a trilobal shape, a diamond shape, a quincuncial shape, and a honeycomb shape. Also, there are no specific limitations on the diameter and particle length of the catalyst particles. It is preferred that the catalyst is in the form of solid cylindrical particles with a diameter of 3 to 3.5 mm and a length of 5 to 10 mm.

In order to solve the third technical problem above, the present disclosure provides an application of the above-described catalyst or the catalyst prepared according to the above-described method in preparation of alkenyl aromatic hydrocarbons by means of dehydrogenation of alkyl aromatic hydrocarbons.

The present disclosure further provides a method for dehydrogenation of an alkyl aromatic hydrocarbon, the method including a step of contacting a feed stream containing an alkyl aromatic hydrocarbon with the above-described catalyst or the catalyst prepared according to the above-described method to obtain a reactant stream containing an alkenyl aromatic hydrocarbon.

According to a preferred implementation of the present disclosure, those skilled in the art can use the application according to the prior art process. The alkyl aromatic hydrocarbon includes at least one of ethylbenzene, methyl ethylbenzene, diethylbenzene or polyalkylbenzene. Preferably, the alkyl aromatic hydrocarbon is diethylbenzene or ethylbenzene. For example, ethylbenzene is used as a raw material, and in the presence of the catalyst, the raw material contacts and reacts with the catalyst so as to produce styrene.

The prepared catalyst is evaluated for activity in an isothermal fixed bed. For the activity evaluation of the catalyst for dehydrogenation of ethylbenzene to styrene, the process is briefly described as follows.

Reaction raw materials are respectively fed into a preheating mixer via a metering pump, and are preheated and mixed into a gaseous state and then enter a reactor. The reactor is heated by an electric heating wire so as to reach a predetermined temperature. The reactant flowing out of the reactor is condensed by water, and then the composition of the reactant is analyzed by means of a gas chromatograph.

The conversion of ethylbenzene and the selectivity of styrene are calculated according to the following formulas:

$$\text{Conversion of ethylbenzene \%} = \frac{\text{Concentration of ethylbenzene before reaction \%(weight) - Concentration of ethylbenzene after reaction \%(weight)}}{\text{Concentration of ethylbenzene before reaction \%(weight)}}$$

$$\text{Selectivity of styrene \%} = \frac{\text{Concentration of styrene produced \%(weight)}}{\text{Concentration of ethylbenzene before reaction \%(weight) - Concentration of ethylbenzene after reaction \%(weight)}}$$

In the present disclosure, an XRD measurement of the catalyst is carried out using D8 advance X-ray powder diffractometer from Bruker, with tube voltage of 40 kV, tube current of 250 mA, Cu target, scanning range of 4° to 70°, scanning speed of 6 (°)/min, and solid detector. In the context of this description, in the XRD data of the catalyst, I represents the peak area of a corresponding diffraction peak; I$_0$ represents the peak area of the strongest diffraction peak; and W, M, S, and VS represent the intensity of a diffraction peak, wherein W means weak, M means medium, S means strong, and VS means very strong, which is well known to those skilled in the art. Generally, W is less than 20; M is in a range from 20 to 40; S is in a range from 40 to 70; and VS is greater than 70.

In the technical solution of the present disclosure, adding potassium in an iron-potassium-cerium-molybdenum-calcium system in different steps, and in the as-prepared catalyst, a compound containing iron and potassium contains both a $K_2Fe_{10}O_{16}$ phase and a $K_2Fe_{22}O_{34}$ phase. Thus, the catalyst has the characteristic of high activity. When the catalyst of the present disclosure is used in a reaction for dehydrogenation of ethylbenzene to styrene under the conditions of reaction pressure of -55 kPa, liquid space velocity of 1.0 h$^{-1}$, temperature of 620°C, water vapor/ethylbenzene (weight ratio) of 1.2, a conversion can reach 81.2% and a selectivity can reach 95.1%, and thus good technical effects are achieved.

## Brief Description of the Drawings

Fig. 1 is an XRD spectrum of a catalyst prepared in Example 1 of the present disclosure.

## Detailed Description of the Disclosure

The present disclosure will be further described with reference to embodiments.

Example 1

Red iron oxide equivalent to 59.74 parts of $Fe_2O_3$, yellow iron oxide equivalent to 14.94 parts of $Fe_2O_3$, potassium carbonate equivalent to 8.51 parts of $K_2O$, cerium oxalate equivalent to 10.62 parts of $CeO_2$, ammonium molybdate equivalent to 1.25 parts of $MoO_3$, calcium carbonate equivalent to 1.29 parts of CaO, and 5.0 parts of sodium carboxymethyl cellulose were stirred in a kneader for 1.5 hours so as to obtain a catalyst precursor I. Potassium carbonate equivalent to 3.65 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 350°C for 6 hours, and at 850°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The XRD spectrum of the catalyst is shown in Fig. 1. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Fig. 1 is the XRD spectrum of the catalyst prepared in Example 1. As can be seen from Fig. 1, characteristic diffraction peak of $\alpha$-$Fe_2O_3$ phase at $2\theta$ of 24.14°, 33.15°, 40.85°, 49.48°, and 63.99° do not appear, indicating that the catalyst does not contain an $\alpha$-$Fe_2O_3$ phase. In Fig. 1, diffraction peaks appearing at $2\theta$ of 28.55°, 33.08°, 47.48°, and 56.34° are $CeO_2$ characteristic diffraction peaks, and the remaining diffraction peaks are $K_2Fe_{22}O_{34}$ and $K_2Fe_{10}O_{16}$ characteristic diffraction peaks. This indicates that, iron and potassium in the catalyst completely formed a compound containing iron and potassium, and the compound containing iron and potassium consists of a $K_2Fe_{10}O_{16}$ phase and a $K_2Fe_{22}O_{34}$ phase.

Example 2

Red iron oxide equivalent to 59.74 parts of $Fe_2O_3$, yellow iron oxide equivalent to 14.94 parts of $Fe_2O_3$, potassium carbonate equivalent to 7.30 parts of $K_2O$, cerium oxalate equivalent to 10.62 parts of $CeO_2$, ammonium molybdate equivalent to 1.25 parts of $MoO_3$, calcium carbonate equivalent to 1.29 parts of CaO, and 5.0 parts of sodium carboxymethyl cellulose were stirred in a kneader for 1.5 hours so as to obtain a catalyst precursor I. Potassium carbonate equivalent to 4.86 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 350°C for 6 hours, and at 850°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 3

Red iron oxide equivalent to 59.74 parts of $Fe_2O_3$, yellow iron oxide equivalent to 14.94 parts of $Fe_2O_3$, potassium

carbonate equivalent to 9.73 parts of $K_2O$, cerium oxalate equivalent to 10.62 parts of $CeO_2$, ammonium molybdate equivalent to 1.25 parts of $MoO_3$, calcium carbonate equivalent to 1.29 parts of CaO, and 5.0 parts of sodium carboxymethyl cellulose were stirred in a kneader for 1.5 hours so as to obtain a catalyst precursor I. Potassium carbonate equivalent to 2.43 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 350°C for 6 hours, and at 850°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 4

Red iron oxide equivalent to 59.74 parts of $Fe_2O_3$, yellow iron oxide equivalent to 14.94 parts of $Fe_2O_3$, potassium carbonate equivalent to 10.34 parts of $K_2O$, cerium oxalate equivalent to 10.62 parts of $CeO_2$, ammonium molybdate equivalent to 1.25 parts of $MoO_3$, calcium carbonate equivalent to 1.29 parts of CaO, and 5.0 parts of sodium carboxymethyl cellulose were stirred in a kneader for 1.5 hours so as to obtain a catalyst precursor I. Potassium carbonate equivalent to 1.82 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 350°C for 6 hours, and at 850°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Comparative Example 1

Except that K is all added by dry mixing, the method for preparing catalyst and catalyst evaluation conditions are the same as those in Example 1, and are specifically as follows.

Red iron oxide equivalent to 59.74 parts of $Fe_2O_3$, yellow iron oxide equivalent to 14.94 parts of $Fe_2O_3$, potassium carbonate equivalent to 12.16 parts of $K_2O$, cerium oxalate equivalent to 10.62 parts of $CeO_2$, ammonium molybdate equivalent to 1.25 parts of $MoO_3$, calcium carbonate equivalent to 1.29 parts of CaO, and 5.0 parts of sodium carboxymethyl cellulose were stirred in a kneader for 1.5 hours, added into deionized water accounting for 23.5% of the total weight of the catalyst raw materials, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 350°C for 6 hours, and at 850°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Comparative Example 2

Except that K is all added by being dissolved with water, the method for preparing catalyst and catalyst evaluation conditions are the same as those in Example 1, and are specifically as follows.

Red iron oxide equivalent to 59.74 parts of $Fe_2O_3$, yellow iron oxide equivalent to 14.94 parts of $Fe_2O_3$, cerium oxalate equivalent to 10.62 parts of $CeO_2$, ammonium molybdate equivalent to 1.25 parts of $MoO_3$, calcium carbonate equivalent to 1.29 parts of CaO, and 5.0 parts of sodium carboxymethyl cellulose were stirred in a kneader for 1.5 hours so as to obtain a catalyst precursor I. Potassium carbonate equivalent to 12.16 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 350°C for 6 hours, and at 850°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Comparative Example 3

Except for the difference in the amount of dry-mixed K in the total amount of K, the method for preparing catalyst and catalyst evaluation conditions are the same as those in Example 1, and are specifically as follows.

Red iron oxide equivalent to 59.74 parts of $Fe_2O_3$, yellow iron oxide equivalent to 14.94 parts of $Fe_2O_3$, potassium carbonate equivalent to 2.43 parts of $K_2O$, cerium oxalate equivalent to 10.62 parts of $CeO_2$, ammonium molybdate equivalent to 1.25 parts of $MoO_3$, calcium carbonate equivalent to 1.29 parts of CaO, and 5.0 parts of sodium carboxymethyl cellulose were stirred in a kneader for 1.5 hours so as to obtain a catalyst precursor I. Potassium carbonate equivalent to 9.73 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 350°C for 6 hours, and at 850°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Comparative Example 4

Except for the difference in the amount of dry-mixed K in the total amount of K, the method for preparing catalyst and catalyst evaluation conditions are the same as those in Example 1, and are specifically as follows.

Red iron oxide equivalent to 59.74 parts of $Fe_2O_3$, yellow iron oxide equivalent to 14.94 parts of $Fe_2O_3$, potassium carbonate equivalent to 11.55 parts of $K_2O$, cerium oxalate equivalent to 10.62 parts of $CeO_2$, ammonium molybdate equivalent to 1.25 parts of $MoO_3$, calcium carbonate equivalent to 1.29 parts of CaO, and 5.0 parts of sodium carboxymethyl cellulose were stirred in a kneader for 1.5 hours so as to obtain a catalyst precursor I. Potassium carbonate equivalent to 0.61 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 350°C for 6 hours, and at 850°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Comparative Example 5

Except for the difference in the amount of dry-mixed K in the total amount of K, the method for preparing catalyst and catalyst evaluation conditions are the same as those in Example 1, and are specifically as follows.

Red iron oxide equivalent to 59.74 parts of $Fe_2O_3$, yellow iron oxide equivalent to 14.94 parts of $Fe_2O_3$, potassium carbonate equivalent to 6.08 parts of $K_2O$, cerium oxalate equivalent to 10.62 parts of $CeO_2$, ammonium molybdate equivalent to 1.25 parts of $MoO_3$, calcium carbonate equivalent to 1.29 parts of $CaO$, and 5.0 parts of sodium carboxymethyl cellulose were stirred in a kneader for 1.5 hours so as to obtain a catalyst precursor I. Potassium carbonate equivalent to 6.08 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 350°C for 6 hours, and at 850°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 h$^{-1}$, temperature of 620°C, water vapor/ethylbenzene (weight ratio) of 1.2. The test results are listed in Table 2.

Example 5

Red iron oxide equivalent to 57.61 parts of $Fe_2O_3$, yellow iron oxide equivalent to 14.40 parts of $Fe_2O_3$, potassium carbonate equivalent to 7.67 parts of $K_2O$, cerium oxalate equivalent to 11.34 parts of $CeO_2$, ammonium molybdate equivalent to 1.75 parts of $MoO_3$, calcium hydroxide equivalent to 2.12 parts of $CaO$, and 5.5 parts of sodium carboxymethyl cellulose were stirred in a kneader for 1.3 hours to obtain a catalyst precursor I. Potassium carbonate equivalent to 5.11 parts of $K_2O$ was dissolved with deionized water accounting for 22.7% of the total weight of the catalyst raw materials, then added to the catalyst precursor I, wet-kneaded for 0.8 hours, taken out, and extruded into particles with a diameter of 3 mm and a length of 5 mm. The particles thus obtained were put into an oven, baked at 40°C for 6 hours, and 110°C for 12 hours, and then calcined at 200°C for 12 hours, and at 900°C for 4 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 h$^{-1}$, temperature of 620°C, water vapor/ethylbenzene (weight ratio) of 1.2. The test results are listed in Table 2.

Example 6

Red iron oxide equivalent to 63.96 parts of $Fe_2O_3$, yellow iron oxide equivalent to 15.99 parts of $Fe_2O_3$, potassium carbonate equivalent to 3.91 parts of $K_2O$, cerium oxalate equivalent to 13.01 parts of $CeO_2$, ammonium molybdate equivalent to 0.51 parts of $MoO_3$, calcium hydroxide equivalent to 0.52 parts of $CaO$, and 5.1 parts of sodium carboxymethyl cellulose were stirred in a kneader for 1.5 hours so as to obtain a catalyst precursor I. Potassium carbonate equivalent to 2.10 parts of $K_2O$ was dissolved with deionized water accounting for 23.5% of the total weight of the catalyst raw materials, then added to the catalyst precursor I, wet-kneaded for 0.8 hours, taken out, and extruded into particles with a diameter of 3 mm and a length of 5 mm. The particles thus obtained were put into an oven, baked at 60°C for 3 hours, and 120°C for 10 hours, and then calcined at 380°C for 6 hours, and at 870°C for 4.5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 h$^{-1}$, temperature of 620°C, water vapor/ethylbenzene (weight ratio) of 1.2. The test results are listed in Table 2.

Example 7

Red iron oxide equivalent to 52.01 parts of $Fe_2O_3$, yellow iron oxide equivalent to 13.00 parts of $Fe_2O_3$, potassium carbonate equivalent to 9.79 parts of $K_2O$, cerium carbonate equivalent to 11.04 parts of $CeO_2$, ammonium molybdate equivalent to 4.98 parts of $MoO_3$, calcium carbonate equivalent to 2.98 parts of CaO, calcium hydroxide equivalent to 2.0 parts of CaO, and 5.5 parts of sodium carboxymethyl cellulose were stirred in a kneader for 2 hours so as to obtain a catalyst precursor I. Potassium carbonate equivalent to 4.20 parts of $K_2O$ was dissolved with deionized water accounting for 23.5% of the total weight of the catalyst raw materials, then added to the catalyst precursor I, wet-kneaded for 0.7 hours, taken out, and extruded into particles with a diameter of 3 mm and a length of 5 mm. The particles thus obtained were put into an oven, baked at 75°C for 2 hours, and 130°C for 4 hours, and then calcined at 400°C for 5 hours, and at 750°C for 8 hours to obtain an as-prepared catalyst. The components of the catalyst are listed in Table 1.
An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.
100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 8

Red iron oxide equivalent to 58.46 parts of $Fe_2O_3$, yellow iron oxide equivalent to 14.61 parts of $Fe_2O_3$, potassium carbonate equivalent to 9.01 parts of $K_2O$, cerium oxalate equivalent to 9.01 parts of $CeO_2$, ammonium molybdate equivalent to 3.56 parts of $MoO_3$, calcium carbonate equivalent to 2.34 parts of CaO, and 5.4 parts of sodium carboxymethyl cellulose were stirred in a kneader for 1.8 hours so as to obtain a catalyst precursor I. Potassium carbonate equivalent to 3.01 parts of $K_2O$ was dissolved with deionized water accounting for 23.5% of the total weight of the catalyst raw materials, then added to the catalyst precursor I, wet-kneaded for 0.9 hours, taken out, and extruded into particles with a diameter of 3 mm and a length of 5 mm. The particles thus obtained were put into an oven, baked at 55°C for 4.5 hours, and 120°C for 8 hours, and then calcined at 360°C for 9 hours, and at 830°C for 4.5 hours to obtain an as-prepared catalyst. The components of the catalyst are listed in Table 1.
An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.
100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 9

Red iron oxide equivalent to 60.54 parts of $Fe_2O_3$, yellow iron oxide equivalent to 15.14 parts of $Fe_2O_3$, potassium carbonate equivalent to 8.90 parts of $K_2O$, cerium oxalate equivalent to 11.07 parts of $CeO_2$, ammonium molybdate equivalent to 0.98 parts of $MoO_3$, calcium carbonate equivalent to 1.15 parts of CaO, and 4.9 parts of sesbania powder were stirred in a kneader for 1.1 hours so as to obtain a catalyst precursor I. Potassium carbonate equivalent to 2.22 parts of $K_2O$ was dissolved with deionized water accounting for 23.5% of the total weight of the catalyst raw materials, then added to the catalyst precursor I, wet-kneaded for 0.5 hours, taken out, and extruded into particles with a diameter of 3 mm and a length of 5 mm. The particles thus obtained were put into an oven, baked at 55°C for 4.5 hours, and 120°C for 10 hours, and then calcined at 360°C for 6 hours, and at 880°C for 4 hours to obtain an as-prepared catalyst. The components of the catalyst are listed in Table 1.
An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.
100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 10

Red iron oxide equivalent to 53.38 parts of $Fe_2O_3$, yellow iron oxide equivalent to 13.34 parts of $Fe_2O_3$, potassium carbonate equivalent to 11.03 parts of $K_2O$, cerium carbonate equivalent to 12.86 parts of $CeO_2$, ammonium molybdate equivalent to 3.28 parts of $MoO_3$, calcium carbonate equivalent to 4.11 parts of CaO, 0.05 parts of $TiO_2$, 3.1 parts of sodium carboxymethyl cellulose, and 2.2 parts of sesbania powder were stirred in a kneader for 1.2 hours so as to obtain a catalyst precursor I. Potassium carbonate equivalent to 1.95 parts of $K_2O$ was dissolved with deionized water accounting for 23.5% of the total weight of the catalyst raw materials, then added to the catalyst precursor I, wet-kneaded for 0.7 hours, taken out, and extruded into particles with a diameter of 3 mm and a length of 5 mm. The particles thus obtained were put into an oven, baked at 50°C for 4.5 hours, and 120°C for 10 hours, and then calcined at 360°C for 6 hours, and at 800°C for 5 hours to obtain an as-prepared catalyst. The components of the catalyst are listed in Table 1.
An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.
100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 11

Red iron oxide equivalent to 56.34 parts of $Fe_2O_3$, yellow iron oxide equivalent to 18.87 parts of $Fe_2O_3$, potassium carbonate equivalent to 7.67 parts of $K_2O$, cerium oxalate equivalent to 12.51 parts of $CeO_2$, ammonium molybdate equivalent to 2.03 parts of $MoO_3$, calcium carbonate equivalent to 1.82 parts of CaO, and 5.8 parts of sodium carboxymethyl cellulose were stirred in a kneader for 1.3 hours so as to obtain a catalyst precursor I. Potassium carbonate equivalent to 0.85 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 55°C for 4.5 hours, and 120°C for 10 hours, and then calcined at 370°C for 6 hours, and at 860°C for 5 hours to obtain an as-prepared catalyst. The components of the catalyst are listed in Table 1.
An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.
100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 12

Red iron oxide equivalent to 59.24 parts of $Fe_2O_3$, yellow iron oxide equivalent to 19.75 parts of $Fe_2O_3$, potassium carbonate equivalent to 7.71 parts of $K_2O$, cerium oxalate equivalent to 8.05 parts of $CeO_2$, ammonium molybdate equivalent to 1.24 parts of $MoO_3$, calcium carbonate equivalent to 1.01 parts of CaO, and 5.6 parts of sodium carboxymethyl cellulose were stirred in a kneader for 1.3 hours so as to obtain a catalyst precursor I. Potassium carbonate equivalent to 3.00 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 55°C for 4.5 hours, and 120°C for 8 hours, and then calcined at 390°C for 5 hours, and at 810°C for 5 hours to obtain an as-prepared catalyst. The components of the catalyst are listed in Table 1.
An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.
100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 13

Red iron oxide equivalent to 54.34 parts of $Fe_2O_3$, yellow iron oxide equivalent to 18.12 parts of $Fe_2O_3$, potassium carbonate equivalent to 7.23 parts of $K_2O$, cerium oxalate equivalent to 13.46 parts of $CeO_2$, ammonium molybdate equivalent to 2.01 parts of $MoO_3$, calcium carbonate equivalent to 2.56 parts of CaO, and 5.3 parts of sodium carboxymethyl cellulose were stirred in a kneader for 1.5 hours so as to obtain a catalyst precursor I. Potassium carbonate equivalent to 2.28 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 360°C for 6 hours, and at 820°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 14

Red iron oxide equivalent to 56.49 parts of $Fe_2O_3$, yellow iron oxide equivalent to 18.83 parts of $Fe_2O_3$, potassium carbonate equivalent to 6.91 parts of $K_2O$, cerium oxalate equivalent to 10.62 parts of $CeO_2$, ammonium molybdate equivalent to 2.21 parts of $MoO_3$, calcium carbonate equivalent to 1.69 parts of CaO, and 5.1 parts of graphite were stirred in a kneader for 1.5 hours so as to obtain a catalyst precursor I. Potassium carbonate equivalent to 3.25 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 340°C for 7 hours, and at 880°C for 4 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 15

Red iron oxide equivalent to 55.23 parts of $Fe_2O_3$, yellow iron oxide equivalent to 18.41 parts of $Fe_2O_3$, potassium carbonate equivalent to 8.74 parts of $K_2O$, cerium oxalate equivalent to 12.43 parts of $CeO_2$, ammonium molybdate equivalent to 1.05 parts of $MoO_3$, calcium carbonate equivalent to 0.21 parts of CaO, and 5.4 parts of sodium carboxymethyl cellulose were stirred in a kneader for 1.5 hours so as to obtain a catalyst precursor I. Potassium carbonate equivalent to 3.93 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 370°C for 6 hours, and at 825°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 16

Red iron oxide equivalent to 56.41 parts of $Fe_2O_3$, yellow iron oxide equivalent to 18.20 parts of $Fe_2O_3$, potassium carbonate equivalent to 9.80 parts of $K_2O$, cerium oxalate equivalent to 12.71 parts of $CeO_2$, ammonium molybdate equivalent to 1.16 parts of $MoO_3$, calcium carbonate equivalent to 1.35 parts of CaO, 0.02 parts of $TiO_2$, and 5.6 parts of sodium carboxymethyl cellulose were stirred in a kneader for 1.5 hours so as to obtain a catalyst precursor I. Potassium carbonate equivalent to 2.15 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 55°C for 3.5 hours, and 120°C for 10 hours, and then calcined at 370°C for 9 hours, and at 810°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 17

Red iron oxide equivalent to 54.82 parts of $Fe_2O_3$, yellow iron oxide equivalent to 18.27 parts of $Fe_2O_3$, potassium carbonate equivalent to 10.47 parts of $K_2O$, cerium oxalate equivalent to 11.35 parts of $CeO_2$, ammonium molybdate equivalent to 1.51 parts of $MoO_3$, calcium carbonate equivalent to 2.02 parts of CaO, and 5.1 parts of sodium carboxymethyl cellulose were stirred in a kneader for 1.5 hours so as to obtain a catalyst precursor I. Potassium carbonate equivalent to 1.56 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 375°C for 6 hours, and at 820°C for 5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 18

Red iron oxide equivalent to 54.71 parts of $Fe_2O_3$, yellow iron oxide equivalent to 18.23 parts of $Fe_2O_3$, potassium carbonate equivalent to 8.38 parts of $K_2O$, cerium oxalate equivalent to 11.38 parts of $CeO_2$, ammonium molybdate equivalent to 1.52 parts of $MoO_3$, calcium carbonate equivalent to 2.83 parts of CaO, and 5.9 parts of sodium carboxymethyl cellulose were stirred in a kneader for 1.5 hours so as to obtain a catalyst precursor I. Potassium carbonate equivalent to 2.95 parts of $K_2O$ was dissolved with deionized water accounting for 23.5% of the total weight of the catalyst raw materials, then added to the catalyst precursor I, wet-kneaded for 0.6 hours, taken out, and extruded into particles with a diameter of 3 mm and a length of 5 mm. The particles thus obtained were put into an oven, baked at 50°C for 4.5 hours, and 120°C for 10 hours, and then calcined at 385°C for 6.5 hours, and at 815°C for 5.5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 19

Red iron oxide equivalent to 59.04 parts of $Fe_2O_3$, yellow iron oxide equivalent to 19.68 parts of $Fe_2O_3$, potassium carbonate equivalent to 3.61 parts of $K_2O$, cerium oxalate equivalent to 8.10 parts of $CeO_2$, ammonium molybdate equivalent to 3.91 parts of $MoO_3$, calcium hydroxide equivalent to 3.26 parts of CaO, and 5.2 parts of sodium carboxymethyl cellulose were stirred in a kneader for 1.6 hours so as to obtain a catalyst precursor I. Potassium carbonate equivalent to 2.40 parts of $K_2O$ was dissolved with deionized water accounting for 23.5% of the total weight of the catalyst raw materials, then added to the catalyst precursor I, wet-kneaded for 0.7 hours, taken out, and extruded into particles with a diameter of 3 mm and a length of 5 mm. The particles thus obtained were put into an oven, baked at 60°C for 3 hours, and 120°C for 10 hours, and then calcined at 380°C for 6 hours, and at 870°C for 4.5 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Example 20

Red iron oxide equivalent to 50.69 parts of $Fe_2O_3$, yellow iron oxide equivalent to 16.90 parts of $Fe_2O_3$, potassium carbonate equivalent to 12.59 parts of $K_2O$, cerium carbonate equivalent to 10.37 parts of $CeO_2$, ammonium molybdate equivalent to 3.78 parts of $MoO_3$, calcium carbonate equivalent to 4.27 parts of CaO, and 5.5 parts of sodium carboxymethyl cellulose were stirred in a kneader for 2 hours so as to obtain a catalyst precursor I. Potassium carbonate equivalent to 1.40 parts of $K_2O$ was dissolved with deionized water accounting for 23.5% of the total weight of the catalyst raw materials, then added to the catalyst precursor I, wet-kneaded for 0.7 hours, taken out, and extruded into particles with a diameter of 3 mm and a length of 5 mm. The particles thus obtained were put into an oven, baked at 75°C for 2 hours, and 130°C for 4 hours, and then calcined at 400°C for 5 hours, and at 750°C for 8 hours to obtain an as-prepared catalyst. The components of the catalyst are listed in Table 1.

An XRD measurement was performed on the catalyst. The XRD measurement was carried out using D8 advance X-ray powder diffractometer from Bruker, with tube voltage of 40 kV, tube current of 250 mA, Cu target, scanning range of 4° to 70°, scanning speed of 6 (°)/min, and solid detector. The results of the crystal phase composition of the sample are listed in Table 2.

100 ml of the catalyst was put into a stainless steel tube reactor with an inner diameter of 1" under the conditions of reaction pressure of -55 kPa, liquid space velocity of 1.0 $h^{-1}$, temperature of 620°C, water vapor/ethylbenzene (weight ratio) of 1.2. The test results are listed in Table 2.

Table 1

| Component content (part) | $Fe_2O_3$ | $K_2O_3$ | $CeO_2$ | $MoO_3$ | CaO | $TiO_2$ | Amount of first part of K in total amount of K |
|---|---|---|---|---|---|---|---|
| Example 1 | 74.68 | 12.16 | 10.62 | 1.25 | 1.29 | 0 | 70 |
| Example 2 | 74.68 | 12.16 | 10.62 | 1.25 | 1.29 | 0 | 60 |
| Example 3 | 74.68 | 12.16 | 10.62 | 1.25 | 1.29 | 0 | 80 |
| Example 4 | 74.68 | 12.16 | 10.62 | 1.25 | 1.29 | 0 | 85 |
| Comparative Example 1 | 74.68 | 12.16 | 10.62 | 1.25 | 1.29 | 0 | 100 |
| Comparative Example 2 | 74.68 | 12.16 | 10.62 | 1.25 | 1.29 | 0 | 0 |
| Comparative Example 3 | 74.68 | 12.16 | 10.62 | 1.25 | 1.29 | 0 | 20 |
| Comparative Example 4 | 74.68 | 12.16 | 10.62 | 1.25 | 1.29 | 0 | 95 |
| Comparative Example 5 | 74.68 | 12.16 | 10.62 | 1.25 | 1.29 | 0 | 50 |
| Example 5 | 72.01 | 12.78 | 11.34 | 1.75 | 2.12 | 0 | 60 |
| Example 6 | 79.95 | 6.01 | 13.01 | 0.51 | 0.52 | 0 | 65 |
| Example 7 | 65.01 | 13.99 | 11.04 | 4.98 | 4.98 | 0 | 70 |

(continued)

| Component content (part) | Fe$_2$O$_3$ | K$_2$O$_3$ | CeO$_2$ | MoO$_3$ | CaO | TiO$_2$ | Amount of first part of K in total amount of K |
|---|---|---|---|---|---|---|---|
| Example 8 | 73.07 | 12.02 | 9.01 | 3.56 | 2.34 | 0 | 75 |
| Example 9 | 75.68 | 11.12 | 11.07 | 0.98 | 1.15 | 0 | 80 |
| Example 10 | 66.72 | 12.98 | 12.86 | 3.28 | 4.11 | 0.05 | 85 |
| Example 11 | 75.12 | 8.52 | 12.51 | 2.03 | 1.82 | 0 | 90 |
| Example 12 | 78.99 | 10.71 | 8.05 | 1.24 | 1.01 | 0 | 72 |
| Example 13 | 72.46 | 9.51 | 13.46 | 2.01 | 2.56 | 0 | 76 |
| Example 14 | 75.32 | 10.16 | 10.62 | 2.21 | 1.69 | 0 | 68 |
| Example 15 | 73.64 | 12.67 | 12.43 | 1.05 | 0.21 | 0 | 69 |
| Example 16 | 72.81 | 11.95 | 12.71 | 1.16 | 1.35 | 0.02 | 82 |
| Example 17 | 73.09 | 12.03 | 11.35 | 1.51 | 2.02 | 0 | 87 |
| Example 18 | 72.94 | 11.33 | 11.38 | 1.52 | 2.83 | 0 | 74 |
| Example 19 | 78.72 | 6.01 | 8.10 | 3.91 | 3.26 | 0 | 60 |
| Example 20 | 67.59 | 13.99 | 10.37 | 3.78 | 4.27 | 0 | 90 |

Table 2

| Catalyst | Crystal phase composition | K$_2$Fe$_{10}$O$_{16}$/ K$_2$Fe$_{22}$O$_{34}$ | Conversion % | Selectivity % |
|---|---|---|---|---|
| Example 1 | K$_2$Fe$_{10}$O$_{16}$, K$_2$Fe$_{22}$O$_{34}$ | 1.6 | 80.4 | 94.9 |
| Example 2 | K$_2$Fe$_{10}$O$_{16}$, K$_2$Fe$_{22}$O$_{34}$ | 2.5 | 79.1 | 95.1 |
| Example 3 | K$_2$Fe$_{10}$O$_{16}$, K$_2$Fe$_{22}$O$_{34}$ | 1.5 | 80.3 | 94.8 |
| Example 4 | K$_2$Fe$_{10}$O$_{16}$, K$_2$Fe$_{22}$O$_{34}$ | 1.9 | 80.0 | 94.8 |
| Comparative Example 1 | $\alpha$-Fe$_2$O$_3$, K$_2$Fe$_{22}$O$_{34}$ | - | 76.4 | 95.1 |
| Comparative Example 2 | $\alpha$-Fe$_2$O$_3$, K$_2$Fe$_{22}$O$_{34}$ | - | 75.8 | 95.4 |
| Comparative Example 3 | $\alpha$-Fe$_2$O$_3$, K$_2$Fe$_{10}$O$_{16}$, K$_2$Fe$_{22}$O$_{34}$ | - | 77.2 | 94.4 |
| Comparative Example 4 | $\alpha$-Fe$_2$O$_3$, K$_2$Fe$_{10}$O$_{16}$, K$_2$Fe$_{22}$O$_{34}$ | - | 77.8 | 95.3 |
| Comparative Example 5 | K$_2$Fe$_4$O$_7$, K$_2$Fe$_{10}$O$_{16}$, K$_2$Fe$_{22}$O$_{34}$ | - | 77.5 | 94.4 |
| Example 5 | K$_2$Fe$_{10}$O$_{16}$, K$_2$Fe$_{22}$O$_{34}$ | 2.1 | 80.3 | 94.8 |
| Example 6 | K$_2$Fe$_{10}$O$_{16}$, K$_2$Fe$_{22}$O$_{34}$ | 2.9 | 78.8 | 94.9 |
| Example 7 | K$_2$Fe$_{10}$O$_{16}$, K$_2$Fe$_{22}$O$_{34}$ | 1.1 | 78.4 | 95.1 |
| Example 8 | K$_2$Fe$_{10}$O$_{16}$, K$_2$Fe$_{22}$O$_{34}$ | 1.8 | 79.6 | 95.0 |
| Example 9 | K$_2$Fe$_{10}$O$_{16}$, K$_2$Fe$_{22}$O$_{34}$ | 2.0 | 78.7 | 94.5 |
| Example 10 | K$_2$Fe$_{10}$O$_{16}$, K$_2$Fe$_{22}$O$_{34}$ | 1.8 | 79.3 | 94.8 |
| Example 11 | K$_2$Fe$_{10}$O$_{16}$, K$_2$Fe$_{22}$O$_{34}$ | 1.9 | 79.0 | 95.4 |
| Example 12 | K$_2$Fe$_{10}$O$_{16}$, K$_2$Fe$_{22}$O$_{34}$ | 1.2 | 78.6 | 95.1 |
| Example 13 | K$_2$Fe$_{10}$O$_{16}$, K$_2$Fe$_{22}$O$_{34}$ | 1.7 | 80.4 | 94.8 |
| Example 14 | K$_2$Fe$_{10}$O$_{16}$, K$_2$Fe$_{22}$O$_{34}$ | 1.4 | 79.8 | 95.0 |
| Example 15 | K$_2$Fe$_{10}$O$_{16}$, K$_2$Fe$_{22}$O$_{34}$ | 2.1 | 79.2 | 94.5 |
| Example 16 | K$_2$Fe$_{10}$O$_{16}$, K$_2$Fe$_{22}$O$_{34}$ | 1.6 | 81.2 | 95.1 |
| Example 17 | K$_2$Fe$_{10}$O$_{16}$, K$_2$Fe$_{22}$O$_{34}$ | 1.5 | 80.8 | 95.1 |

(continued)

| Catalyst | Crystal phase composition | $K_2Fe_{10}O_{16}/$ $K_2Fe_{22}O_{34}$ | Conversion % | Selectivity % |
|---|---|---|---|---|
| Example 18 | $K_2Fe_{10}O_{16}$, $K_2Fe_{22}O_{34}$ | 1.3 | 80.4 | 94.8 |
| Example 19 | $K_2Fe_{10}O_{16}$, $K_2Fe_{22}O_{34}$ | 4.2 | 78.2 | 94.9 |
| Example 20 | $K_2Fe_{10}O_{16}$, $K_2Fe_{22}O_{34}$ | 0.8 | 78.0 | 94.8 |

In Examples 1 to 4, the amounts of respective raw materials are the same, but the only difference lies in the addition amount of the first part of the potassium source. As can be seen from the results of Examples 1 to 4, different addition amounts of the first part of the potassium source would change the crystal phase composition of the compound containing iron and potassium in the catalyst. In Comparative Examples 1 and 2, the amounts of respective raw materials are the same as those in Examples 1-4, except that the potassium source is not added in different steps. The catalysts prepared in Comparative Examples 1 and 2 contain a free $\alpha$-$Fe_2O_3$ phase and does not contain a $K_2Fe_{10}O_{16}$ phase, and when such catalysts are used in a reaction for dehydrogenation of an alkyl aromatic hydrocarbon, the conversion of reactant is low. In Comparative Examples 3 to 5, the amounts of respective raw materials are the same as those in Examples 1-4, but the only difference lies in that the amount of the first part of the potassium source is not within the scope of the present disclosure. In addition to a $K_2Fe_{10}O_{16}$ phase and a $K_2Fe_{22}O_{34}$, the catalysts prepared in Comparative Examples 3 to 5 further contain a free $\alpha$-$Fe_2O_3$ phase or other phases of the compound containing iron and potassium, and when such catalysts are used in a reaction for dehydrogenation of an alkyl aromatic hydrocarbon, the conversion of reactant is low. As can be seen from the comparison between Examples 1 to 4 and Comparative Examples 1 to 5, according to the method of the present disclosure, a catalyst containing special phases of a compound containing iron and potassium can be obtained, and when such a catalyst is used in a reaction for dehydrogenation of an alkyl aromatic hydrocarbon, the conversion of reactant is relatively high.

Example 21

A catalyst was prepared according to the method of Example 1. The components and crystal phase composition of the catalyst were the same as those in Example 1.

The obtained catalyst was crushed and sieved, so as to separate catalyst with a particle size of 0.5 to 0.7 mm for use. 13.3 milliliters of catalyst with a particle size of 0.5 to 0.7 mm was put into an isothermal tubular reactor, and the reactor was continuously supplied with 14.6 g/h ethylbenzene and 18.3 g/h deionized water at a reaction temperature of 620°C and an initial pressure of 1 atm. The performance of the catalyst was measured under the condition of a water vapor/ethylbenzene ratio of 1.25 kg/kg or 7.36 mol/mol. The measured conversion of ethylbenzene was 75.1% and the selectivity of styrene was 95.2%.

The conversion of ethylbenzene and the selectivity of styrene are determined by means of the following formulas:

$$\text{Conversion of ethylbenzene (mol\%)}=[(A \times M_f - B \times M_P)/(A \times M_f)] \times 100$$

$$\text{Selectivity of styrene (mol\%)}=[(D \times M_P - C \times M_f)/(A \times M_f - B \times M_P)] \times (M_{EB}/M_{ST}) \times 100$$

wherein:

A represents ethylbenzene concentration at reactor inlet (wt%);
B represents ethylbenzene concentration at reactor outlet (wt%);
C represents styrene concentration at reactor inlet (wt%);
D represents styrene concentration at reactor outlet (wt%);
$M_f$ represents average molar mass of raw materials;
$M_P$ represents average molar mass of products;
$M_{EB}$ represents molar mass of ethylbenzene; and
$M_{ST}$ represents molar mass of styrene.

**Claims**

1. A method for preparing a catalyst for dehydrogenation of an alkyl aromatic hydrocarbon, the method comprising following steps of:

   1) dry-mixing a first part of a potassium source with an iron source, a cerium source, a molybdenum source, a calcium source, and a pore-forming agent so as to obtain a catalyst precursor I;
   2) dissolving a second part of the potassium source with water, and adding the catalyst precursor I to obtain an as-made catalyst,

   the catalyst comprising the following components in weight percentage: (a) 65% to 80% of $Fe_2O_3$; (b) 6% to 14% of $K_2O$; (c) 9% to 13.5% of $CeO_2$; (d) 0.5% to 5% of $MoO_3$; and (e) 0.2% to 5% of CaO; and the catalyst does not contain a free $\alpha$-$Fe_2O_3$ phase,
   wherein by amount of contained $K_2O$, a sum of a weight of the first part of the potassium source and a weight of the second part of the potassium source is a total weight of a required amount of potassium source, and the weight of the first part of the potassium source accounts for 60% to 90% of the total weight of the required amount of potassium source.

2. The method for preparing a catalyst for dehydrogenation of an alkyl aromatic hydrocarbon according to claim 1, wherein the step 1) comprises:
   the iron source is added in the form of at least one of red iron oxide and yellow iron oxide; the first part of the potassium source is added in the form of at least one of a potassium salt and potassium hydroxide; the cerium source is added in the form of a cerium salt; the molybdenum source is added in the form of at least one of a molybdenum salt and an oxide of molybdenum; and the calcium source is added in the form of at least one of a calcium salt, calcium oxide, and calcium hydroxide.

3. The method for preparing a catalyst for dehydrogenation of an alkyl aromatic hydrocarbon according to claim 1 or 2, wherein the step 2) comprises:

   the second part of the potassium source is added in the form of at least one of potassium hydroxide and potassium carbonate, and/or
   the method further comprises following step of :
   3) performing processes of wet-kneading, extruding, forming, drying, and calcinating on the as-made catalyst obtained in the step 2).

4. The method for preparing a catalyst for dehydrogenation of an alkyl aromatic hydrocarbon according to any one of claims 1 to 3, wherein the step 3), a drying temperature is in a range from 45°C to 130°C, and a drying time is in a range from 4 to 24 hours, and/or
   calcinating at 200 to 400 °C for 5 to 12 hours, and then at 750 to 950°C for 3 to 8 hours.

5. The method for preparing a catalyst for dehydrogenation of an alkyl aromatic hydrocarbon according to any one of claims 1 to 4, wherein, the as-prepared catalyst comprises a compound containing iron and potassium, the compound containing iron and potassium consisting of a $K_2Fe_{10}O_{16}$ phase and a $K_2Fe_{22}O_{34}$ phase, and/or

   the compound containing iron and potassium has an X-ray diffraction XRD pattern as shown in the following table:

| 2θ (°) | d- Distance (Å) | Relative Intensity (I/I$_0$ × 100) |
|---|---|---|
| 7.355±0.04 | 12.010±0.07 | VS |
| 14.752±0.03 | 6.000±0.03 | S |
| 18.825±0.16 | 4.710±0.05 | W |
| 19.846±0.07 | 4.470±0.03 | W |
| 29.837±0.07 | 2.992±0.04 | S |
| 31.566±0.16 | 2.832±0.03 | M |
| 32.460±0.07 | 2.756±0.02 | W |

(continued)

| 2θ (°) | d- Distance (Å) | Relative Intensity (I/I$_0$ × 100) |
|---|---|---|
| 33.705±0.14 | 2.657±0.03 | S |
| 34.854±0.05 | 2.572±0.02 | M |
| 37.767±0.10 | 2.380±0.02 | M |
| 41.825±0.10 | 2.158±0.02 | W |
| 43.253±0.13 | 2.090±0.03 | M |
| 44.232±0.18 | 2.046±0.03 | W |
| 62.351±0.15 | 1.488±0.02 | M |

preferably, wherein the X-ray diffraction pattern further comprises X-ray diffraction peaks as shown in the following table:

| 2θ (°) | d- Distance (Å) | Relative Intensity (I/I$_0$ × 100) |
|---|---|---|
| 17.868±0.08 | 4.960±0.03 | W |
| 30.346±0.03 | 2.943±0.02 | M |
| 36.342±0.11 | 2.470±0.03 | W-M |
| 39.929±0.18 | 2.256±0.03 | W |
| 53.921±0.16 | 1.699±0.03 | M |
| 55.549±0.06 | 1.653±0.02 | W |
| 63.154±0.07 | 1.471±0.02 | W |

6. The method for preparing a catalyst for dehydrogenation of an alkyl aromatic hydrocarbon according to any one of claims 1 to 5, wherein a mass ratio of the $K_2Fe_{10}O_{16}$ phase to the $K_2Fe_{22}O_{34}$ phase in the compound containing iron and potassium is in a range from 1.1 to 3.3, preferably in a range from 1.2 to 2.0.

7. A method for dehydrogenation of an alkyl aromatic hydrocarbon, the method comprising a step of contacting a feed stream containing an alkyl aromatic hydrocarbon with the catalyst prepared according to the method of any one of claims 1 to 6 to obtain a reactant stream containing an alkenyl aromatic hydrocarbon.

8. The method for dehydrogenation of an alkyl aromatic hydrocarbon according to claim 7, wherein the alkyl aromatic hydrocarbon comprises at least one of ethylbenzene, methyl ethylbenzene, diethylbenzene or polyalkylbenzene.

9. The method for dehydrogenation of an alkyl aromatic hydrocarbon according to claim 7 or 8, wherein the alkyl aromatic hydrocarbon is diethylbenzene or ethylbenzene.

**Patentansprüche**

1. Verfahren zur Herstellung eines Katalysators zur Dehydrierung eines Alkyl-aromatischen Kohlenwasserstoffs, wobei das Verfahren die folgenden Schritte umfasst:

   1) Trockenmischen eines ersten Teils einer Kalium-Quelle mit einer Eisen-Quelle, einer Cer-Quelle, einer Molybdän-Quelle, einer Calcium-Quelle und einem Porenbildungsmittel, um einen Katalysatorvorläufer I zu erhalten;
   2) Auflösen eines zweiten Teils der Kalium-Quelle in Wasser und Zugabe des Katalysatorvorläufers I, um einen so hergestellten Katalysator zu erhalten,

   wobei der Katalysator die folgenden Komponenten in Gewichtsprozent umfasst: (a) 65% bis 80% $Fe_2O_3$; (b) 6% bis 14% $K_2O$; (c) 9% bis 13,5% $CeO_2$; (d) 0,5% bis 5% $MoO_3$; und (e) 0,2% bis 5% CaO; und der

Katalysator keine freie $\alpha$-Fe$_2$O$_3$-Phase enthält,

wobei hinsichtlich der Menge an enthaltenem K$_2$O die Summe aus dem Gewicht des ersten Teils der Kalium-Quelle und dem Gewicht des zweiten Teils der Kalium-Quelle das Gesamtgewicht der erforderlichen Menge an Kalium-Quelle darstellt und das Gewicht des ersten Teils der Kalium-Quelle 60% bis 90% des Gesamtgewichts der erforderlichen Menge an Kalium-Quelle ausmacht.

2. Verfahren zur Herstellung eines Katalysators zur Dehydrierung eines Alkyl-aromatischen Kohlenwasserstoffs gemäß Anspruch 1, wobei der Schritt 1) umfasst:

   die Eisen-Quelle wird in Form von mindestens einem aus rotem Eisenoxid und gelbem Eisenoxid zugegeben; der erste Teil der Kalium-Quelle wird in Form von mindestens einem aus Kaliumsalz und Kaliumhydroxid zugegeben; die Cer-Quelle wird in Form eines Cersalzes zugegeben; die Molybdän-Quelle wird in Form von mindestens einem aus Molybdänsalz und Molybdänoxid zugegeben; und die Calcium-Quelle wird in Form von mindestens einem aus Calciumsalz, Calciumoxid und Calciumhydroxid zugegeben.

3. Verfahren zur Herstellung eines Katalysators zur Dehydrierung eines Alkyl-aromatischen Kohlenwasserstoffs gemäß Anspruch 1 oder 2, wobei der Schritt 2) umfasst:

   der zweite Teil der Kalium-Quelle wird in Form von mindestens einem aus Kaliumhydroxid und Kaliumcarbonat zugegeben, und/oder

   das Verfahren umfasst ferner den folgenden Schritt:
   3) Durchführen der Verfahren Nasskneten, Extrudieren, Formen, Trocknen und Kalzinieren an dem in Schritt 2) erhaltenen, so hergestellten Katalysator.

4. Verfahren zur Herstellung eines Katalysators zur Dehydrierung eines Alkyl-aromatischen Kohlenwasserstoffs gemäß mindestens einem der Ansprüche 1 bis 3, wobei der Schritt 3) eine Trocknungstemperatur im Bereich von 45°C bis 130°C und eine Trocknungszeit im Bereich von 4 bis 24 Stunden umfasst und/oder

   Kalzinieren bei 200 bis 400°C für 5 bis 12 Stunden und anschließend bei 750 bis 950°C für 3 bis 8 Stunden.

5. Verfahren zur Herstellung eines Katalysators zur Dehydrierung eines Alkyl-aromatischen Kohlenwasserstoffs gemäß mindestens einem der Ansprüche 1 bis 4, wobei der so hergestellte Katalysator eine Verbindung umfasst, die Eisen und Kalium enthält, wobei die Eisen und Kalium enthaltende Verbindung aus einer K$_2$Fe$_{10}$O$_{16}$-Phase und einer K$_2$Fe$_{22}$O$_{34}$-Phase besteht, und/oder

   die Eisen und Kalium enthaltende Verbindung ein Röntgenbeugungsmuster (XRD) aufweist, wie in der folgenden Tabelle gezeigt:

| 2θ (°) | d-Abstand (Å) | Relative Intensität (I/I$_0$x100) |
|---|---|---|
| 7.355±0.04 | 12.010±0.07 | VS |
| 14.752±0.03 | 6.000±0.03 | S |
| 18.825±0.16 | 4.710±0.05 | W |
| 19.846±0.07 | 4.470±0.03 | W |
| 29.837±0.07 | 2.992±0.04 | S |
| 31.566±0.16 | 2.832±0.03 | M |
| 32.460±0.07 | 2.756±0.02 | W |
| 33.705±0.14 | 2.657±0.03 | S |
| 34.854±0.05 | 2.572±0.02 | M |
| 37.767±0.10 | 2.380±0.02 | M |
| 41.825±0.10 | 2.158±0.02 | W |
| 43.253±0.13 | 2.090±0.03 | M |
| 44.232±0.18 | 2.046±0.03 | W |
| 62.351±0.15 | 1.488±0.02 | M |

vorzugsweise, wobei das Röntgenbeugungsmuster ferner Röntgenbeugungspeaks umfasst, wie in der folgenden Tabelle gezeigt:

| $2\theta$ (°) | d-Abstand (Å) | Relative Intensität ($I/I_0\times100$) |
|---|---|---|
| 17.868±0.08 | 4.960±0.03 | W |
| 30.346±0.03 | 2.943±0.02 | M |
| 36.342±0.11 | 2.470±0.03 | W-M |
| 39.929±0.18 | 2.256±0.03 | W |
| 53.921±0.16 | 1.699±0.03 | M |
| 55.549±0.06 | 1.653±0.02 | W |
| 63.154±0.07 | 1.471±0.02 | W |

6. Verfahren zur Herstellung eines Katalysators zur Dehydrierung eines Alkyl-aromatischen Kohlenwasserstoffs gemäß mindestens einem der Ansprüche 1 bis 5, wobei das Massenverhältnis der $K_2Fe_{10}O_{16}$-Phase zur $K_2Fe_{22}O_{34}$-Phase in der Eisen und Kalium enthaltenden Verbindung in einem Bereich von 1,1 bis 3,3, vorzugsweise in einem Bereich von 1,2 bis 2,0 liegt.

7. Verfahren zur Dehydrierung eines Alkyl-aromatischen Kohlenwasserstoffs, wobei das Verfahren einen Schritt umfasst, bei dem ein Zufuhrstrom, der einen Alkyl-aromatischen Kohlenwasserstoff enthält, mit dem gemäß dem Verfahren gemäß mindestens einem der Ansprüche 1 bis 6 hergestellten Katalysator in Kontakt gebracht wird, um einen Reaktionsstrom zu erhalten, der einen Alkenyl-aromatischen Kohlenwasserstoff enthält.

8. Verfahren zur Dehydrierung eines Alkyl-aromatischen Kohlenwasserstoffs gemäß Anspruch 7, wobei der Alkylaromatische Kohlenwasserstoff mindestens eines umfasst aus Ethylbenzol, Methylethylbenzol, Diethylbenzol oder Polyalkylbenzol.

9. Verfahren zur Dehydrierung eines Alkyl-aromatischen Kohlenwasserstoffs gemäß Anspruch 7 oder 8, wobei der Alkylaromatische Kohlenwasserstoff Diethylbenzol oder Ethylbenzol ist.

**Revendications**

1. Procédé destiné à préparer un catalyseur pour une déshydrogénation d'un hydrocarbure aromatique d'alkyle, le procédé comprenant les étapes suivantes consistant à :

   1) mélanger à sec une première partie d'une source de potassium avec une source de fer, une source de cérium, une source de molybdène, une source de calcium et un agent porogène de façon à obtenir un précurseur de catalyseur I;
   2) dissoudre une seconde partie de la source de potassium avec de l'eau, et ajouter le précurseur de catalyseur I pour obtenir un catalyseur tel qu'il a été fabriqué,

   le catalyseur comprenant les constituants suivants en pourcentage en poids : (a) 65 % à 80 % de $Fe_2O_3$ ; (b) 6 % à 14 % de $K_2O$ ; (c) 9 % à 13,5 % de $CeO_2$ ; (d) 0,5 % à 5 % de $MoO_3$ ; et (e) 0,2 % à 5 % de $CaO$ ; et le catalyseur ne contient pas de phase $\alpha$-$Fe_2O_3$ libre,
   dans lequel par quantité de $K_2O$ contenu, une somme d'un poids de la première partie de la source de potassium et d'un poids de la seconde partie de la source de potassium est un poids total d'une quantité requise de source de potassium et le poids de la première partie de la source de potassium représente 60 % à 90 % du poids total de la quantité requise de source de potassium.

2. Procédé destiné à préparer un catalyseur pour une déshydrogénation d'un hydrocarbure aromatique d'alkyle selon la revendication 1, dans lequel l'étape 1) comprend :
   la source de fer est ajoutée sous la forme d'au moins un oxyde parmi l'oxyde de fer rouge et l'oxyde de fer jaune ; la première partie de la source de potassium est ajoutée sous la forme d'au moins un élément parmi un sel de potassium et l'hydroxyde de potassium ; la source de cérium est ajoutée sous la forme d'un sel de cérium ; la source de molybdène est ajoutée sous la forme d'au moins un élément parmi un sel de molybdène et un oxyde de molybdène ; et la source de calcium est ajoutée sous la forme d'au moins un élément parmi un sel de calcium, l'oxyde de calcium et l'hydroxyde de calcium.

3. Procédé destiné à préparer un catalyseur pour une déshydrogénation d'un hydrocarbure aromatique d'alkyle selon la revendication 1 ou la revendication 2, dans lequel l'étape 2) comprend :

la seconde partie de la source de potassium est ajoutée sous la forme d'au moins un élément parmi l'hydroxyde de potassium et le carbonate de potassium, et/ou
le procédé comprend en outre l'étape suivante consistant à :
3) effectuer des processus de pétrissage à l'état humide, d'extrusion, de formage, de séchage et de calcination sur le catalyseur tel qu'il a été fabriqué obtenu à l'étape 2).

4. Procédé destiné à préparer un catalyseur pour une déshydrogénation d'un hydrocarbure aromatique d'alkyle selon l'une quelconque des revendications 1 à 3, dans lequel, à l'étape 3), une température de séchage est dans une plage de 45 °C à 130 °C et un temps de séchage est dans une plage de 4 à 24 heures, et/ou
une calcination à 200 à 400°C pendant 5 à 12 heures et ensuite à 750 à 950°C pendant 3 à 8 heures.

5. Procédé destiné à préparer un catalyseur pour une déshydrogénation d'un hydrocarbure aromatique d'alkyle selon l'une quelconque des revendications 1 à 4, dans lequel le catalyseur tel qu'il a été préparé comprend un composé contenant du fer et du potassium, le composé contenant du fer et du potassium étant constitué d'une phase $K_2Fe_{10}O_{16}$ et d'une phase $K_2Fe_{22}O_{34}$, et/ou

le composé contenant du fer et du potassium présente un schéma de diffraction des rayons X XRD tel que montré dans le tableau suivant :

| 2θ (°) | distance d (Å) | intensité relative (I/I$_0$ x 100) |
|---|---|---|
| 7,355 $\pm$ 0,04 | 12,010 $\pm$ 0,07 | VS (très forte) |
| 14,752 $\pm$ 0,03 | 6,000 $\pm$ 0,03 | S (forte) |
| 18,825 $\pm$ 0,16 | 4,710 $\pm$ 0,05 | W (faible) |
| 19,846 $\pm$ 0,07 | 4,470 $\pm$ 0,03 | W (faible) |
| 29,837 $\pm$ 0,07 | 2,992 $\pm$ 0,04 | S (forte) |
| 31,566 $\pm$ 0,16 | 2,832 $\pm$ 0,03 | M (moyenne) |
| 32,460 $\pm$ 0,07 | 2,756 $\pm$ 0,02 | W (faible) |
| 33,705 $\pm$ 0,14 | 2,657 $\pm$ 0,03 | S (forte) |
| 34,854 $\pm$ 0,05 | 2,572 $\pm$ 0,02 | M (moyenne) |
| 37,767 $\pm$ 0,10 | 2,380 $\pm$ 0,02 | M (moyenne) |
| 41,825 $\pm$ 0,10 | 2,158 $\pm$ 0,02 | W (faible) |
| 43,253 $\pm$ 0,13 | 2,090 $\pm$ 0,03 | M (moyenne) |
| 44,232 $\pm$ 0,18 | 2,046 $\pm$ 0,03 | W (faible) |
| 62,351 $\pm$ 0,15 | 1,488 $\pm$ 0,02 | M (moyenne) |

de prérérence, dans lequel le schéma de diffraction des rayons X comprend en outre des pics de diffraction des rayons X telles que montrés dans le tableau suivant :

| 2θ (°) | distance d (Å) | intensité relative (I/I$_0$ x 100) |
|---|---|---|
| 17,868 $\pm$ 0,08 | 4,960 $\pm$ 0,03 | W (faible) |
| 30,346 $\pm$ 0,03 | 2,943 $\pm$ 0,02 | M (moyenne) |
| 36,342 $\pm$ 0,11 | 2,470 $\pm$ 0,03 | W-M (faible - moyenne) |

(continued)

| 2θ (°) | distance d (Å) | intensité relative ($I/I_0$ x 100) |
|---|---|---|
| $39{,}929 \pm 0{,}18$ | $2{,}256 \pm 0{,}03$ | W (faible) |
| $53{,}921 \pm 0{,}16$ | $1{,}699 \pm 0{,}03$ | M (moyenne) |
| $55{,}549 \pm 0{,}06$ | $1{,}653 \pm 0{,}02$ | W (faible) |
| $63{,}154 \pm 0{,}07$ | $1{,}471 \pm 0{,}02$ | W (faible) |

6. Procédé destiné à préparer un catalyseur pour une déshydrogénation d'un hydrocarbure aromatique d'alkyle selon l'une quelconque des revendications 1 à 5, dans lequel un rapport en masse de la phase $K_2Fe_{10}O_{16}$ sur la phase $K_2Fe_{22}O_{34}$ dans le composé contenant du fer et du potassium est dans une plage de 1,1 à 3,3, de préférence dans une plage de 1,2 à 2,0.

7. Procédé destiné à la déshydrogénation d'un hydrocarbure aromatique d'alkyle, le procédé comprenant une étape consistant à mettre en contact un courant d'alimentation contenant un hydrocarbure aromatique d'alkyle avec le catalyseur préparé selon le procédé selon l'une quelconque des revendications 1 à 6 pour obtenir un courant de réactif contenant un hydrocarbure aromatique alcényle.

8. Procédé destiné à la déshydrogénation d'un hydrocarbure aromatique d'alkyle selon la revendication 7, dans lequel l'hydrocarbure aromatique d'alkyle comprend au moins un composé parmi l'éthylbenzène, l'éthylbenzène de méthyle, le diéthylbenzène ou le polyalkylbenzène.

9. Procédé destiné à la déshydrogénation d'un hydrocarbure aromatique d'alkyle selon la revendication 7 ou la revendication 8, dans lequel l'hydrocarbure aromatique d'alkyle est le diéthylbenène ou l'éthylbenzène.

**EP 3 868 468 B1**